# EUROPÄISCHE PATENTSCHRIFT

(11) **EP 0 418 450 B1**
(45) Veröffentlichungstag und Bekanntmachung des Hinweises auf die Patenterteilung: **12.05.1993**
(21) Anmeldenummer: 90100121.4
(22) Anmeldetag: 04.01.1990
(51) Int. Cl.: A61L 2/06

(54) **Reinigungs- und Sterilisiermaschine für kleine Gegenstände, wie Verschlusselemente von pharmazeutischen Gefässen**
Cleaning and sterilising unit for small objects such as closure elements for pharmaceutical containers
Machine pour le nettoyage et la stérilisation de petits objets tels que les éléments de fermeture de récipients pharmaceutiques

(30) Priorität: 20.09.1989 DE 3931368
(43) Veröffentlichungstag der Anmeldung: 27.03.1991
(73) Patentinhaber: SMEJA GMBH & CO. KG, D-47638 Straelen (DE)
(72) Erfinder: Wieczorek, Joachim, D-4156 Willich 2 (DE)
(74) Vertreter: Cohausz & Florack Patentanwälte

(56) Entgegenhaltungen:
- DE-A- 2 154 582
- DE-A- 2 739 169
- GB-A- 2 212 386
- GB-A- 2 212 387
- Firmenschrift der Fa. Smefa GmbH & Co. KG: "Pharma-Clean"-Maschinen

## Beschreibung

Die Erfindung bezieht sich auf eine Reinigungs- und Sterilisiermaschine für kleine Gegenstände, wie Verschlußelemente von pharmazeutischen Gefäßen, bestehend aus einem um eine horizontale Achse um 180° in einem Träger schwenkbar gelagerten Behandlungsbehälter, der einen trichterförmigen Oberteil mit einer darin angeordneten, verschließbaren Entladeöffnung aufweist und an dem Zu- und Abfuhrleitungen für mindestens ein Behandlungsmedium angeschlossen sind.

Reinigungs- und Sterilisiermaschinen dieser Art sind bekannt. Bei einer bekannten Reinigungs- und Sterilisiermaschine dieser Art ("Pharma-Clean" -Fa. Smeja) ist der Behandlungsbehälter an einer aus der sterilen Kammer herausführenden Saugleitung angeschlossen, über die der Behandlungsbehälter mit den von außerhalb der sterilen Kammer zuzuführenden, zu behandelnden Gegenstände beladen werden kann. Zum Entladen der behandelten Gegenstände wird der Behandlungsbehälter um 180° verschwenkt. Eine in der sterilen Kammer befindliche Bedienungsperson entfernt von der Entladeöffnung einen Deckel und füllt die sterilisierten Gegenstände in einen unter die Entladeöffnung gefahrenen Behälter. Wegen der Beladung des Behandlungsbehälters über die Saugleitung und der von Hand durch die Bedienungsperson notwendigen Entladung in einer sterilen Kammer ist die Behandlung nicht nur arbeitsaufwendig, sondern erfordert auch einen großen Einrichtungsaufwand.

Aufgabe der Erfindung ist es, eine Reinigungs- und Sterilisiermaschine zu schaffen, die einen vergleichsweise geringen einrichtungstechnischen Aufwand in der sterilen Kammer erforderlich macht, insbesondere bei der die Unterbringung des Behandlungsbehälters in einer sterilen Kammer nicht notwendig ist. Darüber hinaus soll mit der Reinigungs- und Sterilisiermaschine der Belade- und Entladevorgang weitgehend mechanisiert werden. Schließlich besteht eine weitere Aufgabe der Erfindung darin, unter Verwendung der erfindungsgemäßen Reinigungs- und Sterilisiermaschine ein Verfahren zum Reinigen und Sterilisieren kleiner Gegenstände zu schaffen.

Diese Aufgabe wird bei einer Reinigungs- und Sterilisiermaschine der eingangs genannten Art dadurch gelöst, daß die Entladeöffnung durch ein Ventil schließbar und zusätzlich als Beladeöffnung mit einem Kupplungsteil ausgebildet ist, an dem ein Be- und Entladecontainer für die zu behandelnden Gegenstände mit einem entsprechenden Kupplungsteil, das an einer durch ein Ventil verschließbaren Be- und Entladeöffnung in einem trichterförmigen Oberteil des Be- und Entladecontainers angeordnet ist, dicht und tragfähig anschließbar ist.

Die erfindungsgemäße Reinigungs- und Sterilisiermaschine macht nicht länger deren Unterbringung in oder an einer sterilen Kammer notwendig. Derselbe Container, der die zu reinigenden unsterilen Gegenstände aufnimmt, nimmt auch die nach der Behandlung im Behandlungsbehälter gereinigten und sterilisierten Gegenstände auf. Dies ist möglich, weil er nach Anschluß an den Behandlungsbehälter mit diesem nach außen hermetisch abgeschlossen ist und sein Innenraum wie der des Behandlungsbehälters während der Behandlung der Gegenstände gereinigt und steril gemacht wird. Es erübrigt sich auch, die sonst für die Behandlung notwendige, in die sterile Kammer zu dem Behandlungsbehälter führende Saugleitung. Die zu behandelnden Gegenstände fallen nach Verschwenken des Behandlungsbehälters mit dem daran tragfähig befestigten Container aus dem Container in den nun unter dem Container befindlichen Behandlungsbehälter. Umgekehrt wird nach erneutem Verschwenken der Behandlungsbehälter in den Container entleert. Nach Verschließen des mit steriliserten Gegenständen gefüllten Containers wird dieser vom Behandlungsbehälter abgekuppelt und kann zur Verbrauchsstelle der Gegenstände transportiert werden.

Sofern die im Container enthaltenen, sterilisierten Gegenstände in kleinere Einheiten unterteilt werden sollen, ist nach einer Ausgestaltung der Erfindung vorgesehen, daß der Container mit seinem Kupplungsteil an ein entsprechendes Kupplungsteil einer zu einem sterilen Raum führenden Schleuse dicht und tragfähig über Kopf anschließbar ist, wobei in der durch einen Bodendeckel verschließbaren Schleuse kupplungsseitig Reinigungs- und Sterilisierorgane angeordnet sind. Nach Anschließen des Containers läßt sich bei noch verschlossenem Ventil des Containers und verschlossenem Bodendeckel der Schleuse der Innenraum der Schleuse sowie die kontaminierte Seite des Ventils des Containers reinigen und sterilisieren, so daß danach der Bodendeckel geöffnet und mit Betätigung des Ventils des Containers die Entladung der sterilisierten Gegenstände freigegeben wird.

Gegenstand der Erfindung ist ferner ein Verfahren zum Reinigen und Sterilisieren kleiner Gegenstände, wie Verschlußelemente von pharmazeutischen Gefäßen unter Verwendung der erfindungsgemäßen Reinigungs- und Sterilisiermaschine. Das Verfahren ist dadurch gekennzeichnet, daß nach Umfüllen der Gegenstände aus dem Container in den an dem Container dicht angekuppelten Behandlungsbehälter die Behandlung der Gegenstände in dem Behandlungsbehälter und parallel dazu die Reinigung und Sterilisierung des leeren Containers erfolgen und daß danach bei angekuppeltem Behandlungsbehälter die gereinigten und sterilisierten Gegenstände in den Container umgefüllt werden und nach Verschließen des Containers der Container von dem Behandlungsbehälter abgekuppelt und an der zu dem Sterilraum führenden Schleuse angekuppelt wird, über die dann nach Reinigung und Sterilisierung des abgeschlossenen Raums zwischen dem Ventil der Ent- und Beladeöffnung unter der Schleuse die gereinigten und sterilisierten Gegenstände in den Sterilraum entleert werden.

Im folgenden wird die Erfindung anhand einer Zeichnung näher erläutert, die in den einzelnen Figuren die Reinigungs- und Sterilisiermaschine während verschiedener Phasen zwischen der Beladung mit zu sterilisierenden Gegenständen und der Verwendung der Gegenstände in einer Abfüllmaschine darstellt.

Die in nicht sterilisierter Umgebung aufgestellte Reinigungs- und Sterilisiermaschine weist einen Behandlungsbehälter 1 auf, der in einem Träger des Maschinengehäuses um eine horizontale Achse 3 um 180° verschwenkbar gelagert ist. Der Behandlungsbehälter 1 hat einen trichterförmigen Unterteil 4 mit einer durch ein verschwenkbares Klappenverntil 6 verschlossenen Ent- und Beladeöffnung 5. Am Behandlungsbehälter 1 sind Zu- und Abfuhrleitungen 7,8 für Behandlungsmedien angeschlossen. Die Behandlung erfolgt in an sich bekannter Weise mit Wasser, Dampf, Heißluft und gegebenenfalls mit einer Silikondispersion. Da die Behandlung einschließlich der besonderen Führung der Behandlungsmedien im Behandlungsbehälter zum Zwecke einer gründlichen und schonenden Behandlung der Gegenstände bekannt ist, wird im folgenden darauf nur kurz eingegangen.

Die Entladeöffnung 5 weist auf der dem Behälter abgewandten Seite einen Kupplungsteil 9 auf. An diesen Kupplungsteil 9 ist ein die zu reinigenden Gegenstände enthaltender Container 11 mit einem entsprechenden Kupplungsteil 10 dicht und tragfähig anschließbar. Der Container 11 weist ein trichterförmiges Oberteil 13 mit einer Be- und Entladeöffnung auf, die durch ein Klappenvestil 12 verschließbar ist. In den Container 11 mündet eine Leitung 14 mit einer Verteilerdüse 15 für die einzuleitenden Behandlungsmedien Wasser, Dampf, Druckluft. An diese Leitung 14 ist eine Schlauchleitung 16 anschließbar, die von den Medienanschlüssen im Maschinengehäuse 2 kommt.

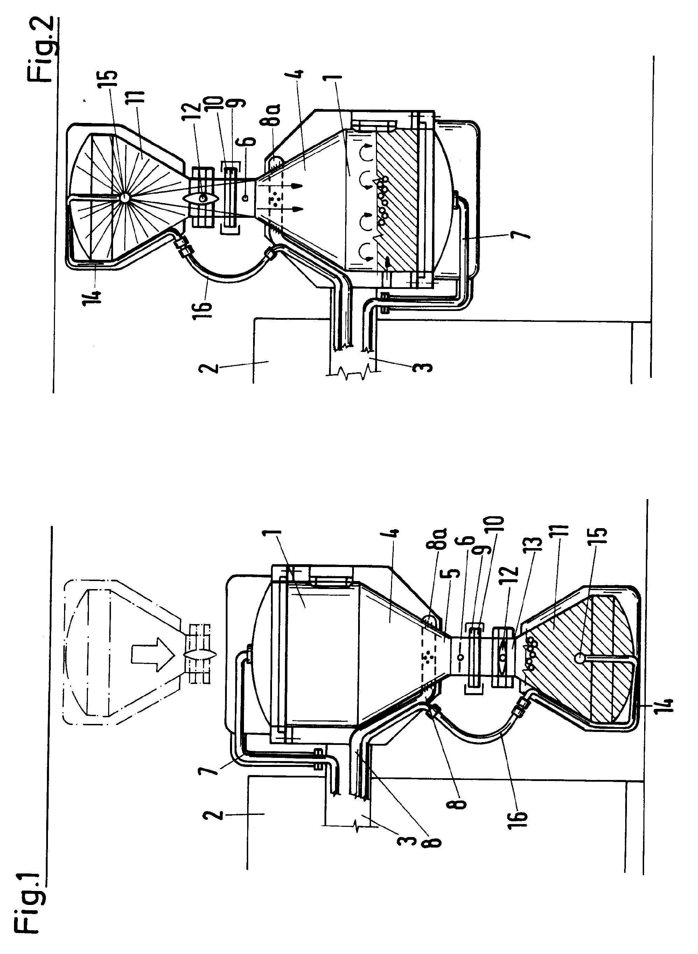

In Figur 1 ist die Reinigungs- und Sterilisiermaschine unmittelbar nach Anschließen des Containers 11 an den Behandlungsbehälter 1 dargestellt. Die Klappenventile 6,12 sind hier noch geschlossen.

In Figur 2 ist die Maschine nach Verschwenken der Einheit 1,11 um 180° dargestellt. Die beiden Klappenventile 6 und 12 sind geöffnet und die zu behandelnden Gegenstände sind schon aus dem Container 11 in den Behandlungsbehälter 1 entleert. Der Container 11 wird durch Besprühen seiner Wände mit Heißwasser, das über die Verteilerdüse 15 zugeführt wird, gereinigt. Dieses Wasser fließt in den Behandlungsbehälter 1, in dem gleichzeitig die Gegenstände im Dampfwirbelbett durch über die Leitung 7 zugeführten Heißdampf gereinigt werden.

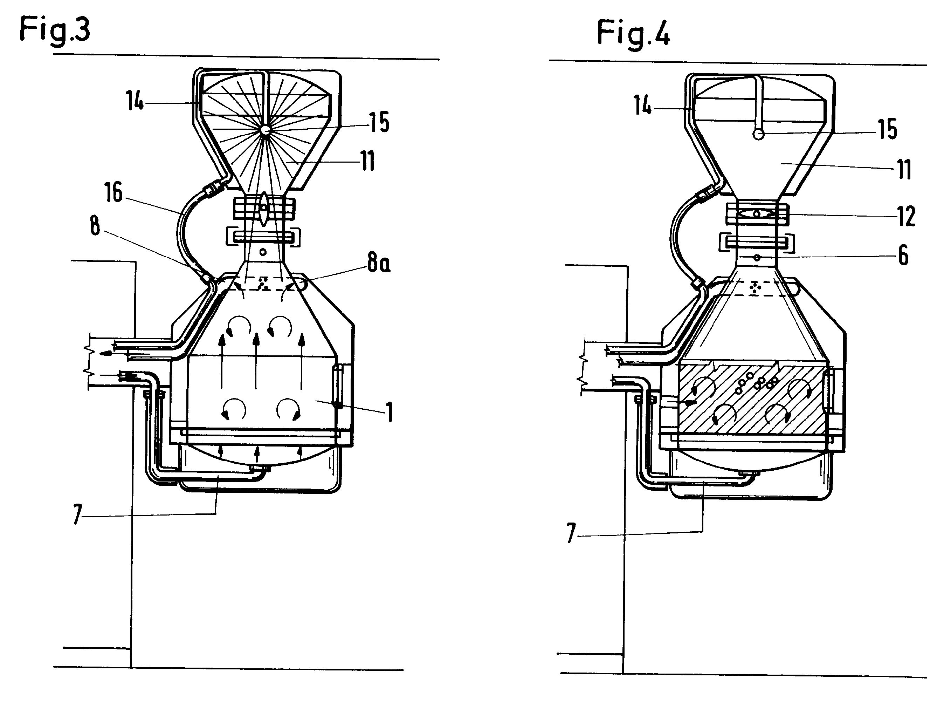

In der Behandlungsstufe gemäß Figur 3 dauert die Reinigung des Containers 11 mit Heißwasser an, während der Behandlungsbehälter 1 mit Wasser gespült wird, das über einen Überlauf 8a über die Leitung 8 abgeführt wird.

In der Behandlungsstufe gemäß Figur 4 sind die Klappenventile 6,12 geschlossen. Die Gegenstände werden bei 100°C im Wirbelbett mit einer Silikondispersion silikonisiert. Nach beendeter Silikonisierung werden die Ventile 6,12 geöffnet und die Silikondispersion durch über die Leitung 14 mit der Verteilerdüse 15 zugeführte Druckluft nach unten gedrückt und über die Leitung 7 abgeleitet.

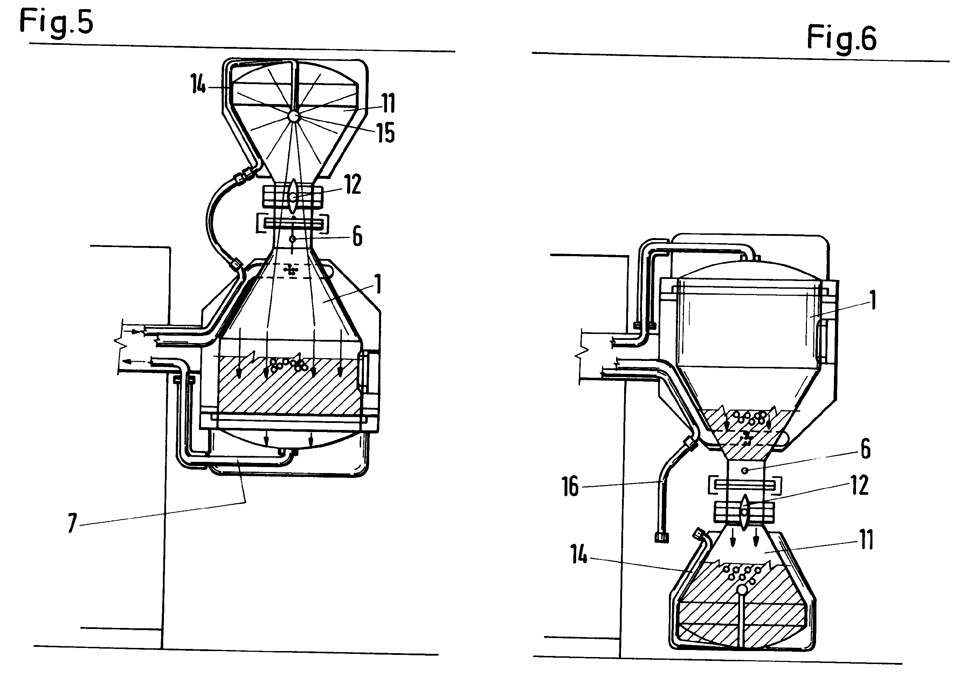

In den Behandlungsphasen gemäß Figur 5 sind die Klappenventile 6,12 geöffnet, und über die Leitung 14 mit der Verteilerdüse 15 wird dem Container 11 und dem Behandlungsbehälter 1 zunächst Reinstdampf zum Sterilisieren der Gegenstände des Behandlungsbehälters 1 und des Containers 11 zugeleitet, der über die untere Leitung 7 abgezogen wird, so daß der Dampfstrom alle Gegenstände erfaßt. Nach der erfolgten Sterilisation bleiben die Ventile 6,12 geöffnet und wird in der anschließenden Phase über die Leitung 14 mit der Verteilerdüse 15 dem Container 11 und dem Behandlungsbehälter 1 zur Trocknung der Gegenstände des Behandlungsbehälters 1 und des Containers 11 steril gefilterte und erhitzte Druckluft zugeführt, die über die Leitung 7 durch Vacuum abgesaugt wird, so daß der Luftstrom alle Gegenstände erfaßt.

Um den Behandlungsbehälter 1 in den Container 11 zu entleeren, wird gemäß Figur 6 die gesamte Einheit wieder 180° verschwenkt. Nachdem alle Gegenstände sich im Container 11 befinden, werden die Klappenventile 6,12 verschlossen, und die mit einem selbst verschließenden Verschluß versehene Leitung 14 von der Leitung 16 getrennt. Der Container 11 wird dann von dem Behandlungsbehälter 1 abgekuppelt. Die gereinigten und sterilisierten Gegenstände befinden sich dann hermetisch verschlossen in dem Container 11, der sich seinerseits in nichtsterilisierter Umgebung befindet.

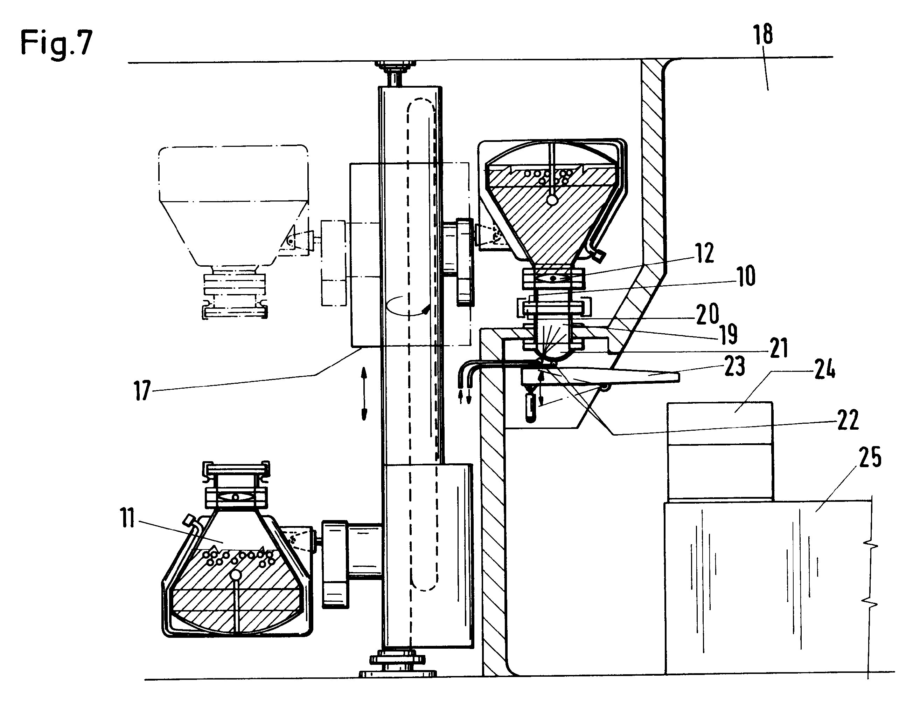

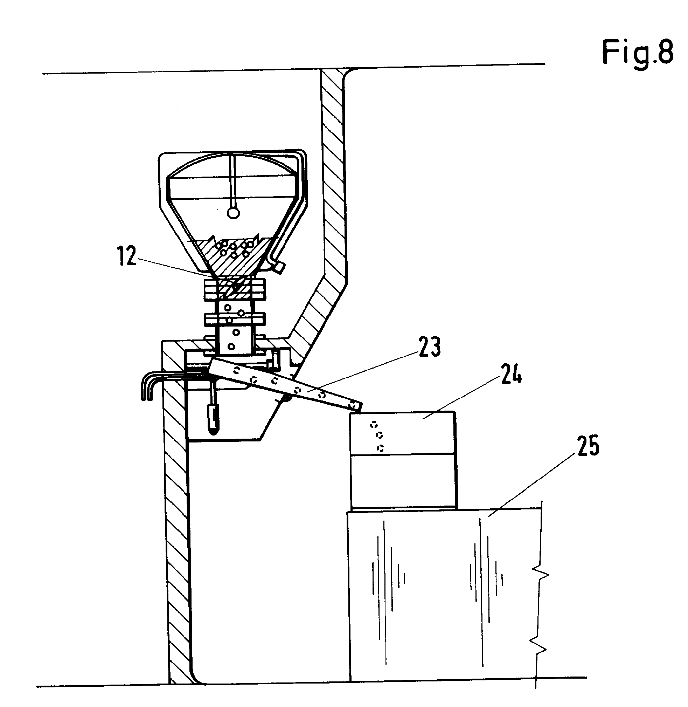

Um die sterilisierten Gegenstände in andere sterile Behälter umzupacken und/oder zu sortieren oder sie als Verschlüsse in einer Abfüllmaschine zu verwenden, ist gemäß Figur 7 und 8 eine Hub- und Drehbühne mit einer Entlade- und Umfüllvorrichtung vorgesehen. Dazu wird der verschlossene Container 11 von der Hub- und Drehbühne 17 angehoben um eine horizontale Achse um 180° und über eine zu einem Sterilraum 18 führende Schleuse 19 verschwenkt, die Teil der Entlade- und Umfüllvorrichtung 23 ist. Die Schleuse 19 weist ein Kupplungsteil 20 auf, das gleich dem Kupplungsteil 9 des Behandlungsbehälters 1 ist, so daß der Container 11 mit seinem Kupplungsteil 10 daran dicht und tragfähig anschließbar ist. Die Schleuse 19 ist zum Sterilraum 18 hin durch einen Bodendeckel 21 verschlossen und mit Reinigungs- und Sterilisierorganen 22 ausgestattet, mit denen nach Anschluß des Containers 11 der Raum zwischen der Ventilklappe 12 und dem Bodendeckel 21 sterilisiert werden kann. Nach dieser Sterilisation kann der Bodendeckel 21 von der Schleuse 19 entfernt werden, wie in Figur 8 dargestellt ist. Nach Öffnen des Klappenventils 12 gelangen die sterilisierten Gegenstände über eine Transportbahn 23 zu einer Sortiermaschine 24 und einer Füllmaschine 25. Das sonst umständliche Umfüllen der steriliserten Gegenstände vom Behandlungsbehälter 1 in einen Container 11 innerhalb eines sterilisierten Raumes durch eine Bedienungsperson entfällt.

## Patentansprüche

1. Reinigungs- und Sterilisiermaschine für kleine Gegenstände, wie Verschlußelemente von pharmazeutischen Gefäßen, bestehend aus einem um eine horizontale Achse (3) um 180° in einem Träger (2) schwenkbar gelagerten Behandlungsbehälter (1), der einen trichterförmigen Unterteil (4) mit einer darin angeordneten, verschließbaren Entladeöffnung (5) aufweist und an dem Zu- und Abfuhrleitungen (7,8) für mindestens ein Behandlungsmedium, wie Wasser, Dampf, Druckluft, angeschlossen sind, **dadurch gekennzeichnet,** daß die Entladeöffnung (5) durch ein Ventil (6) schließbar und zusätzlich als Beladeöffnung mit einem Kupplungsteil (9) ausgebildet ist, an dem ein Be- und Entladecontainer (11) für die zu behandelnden Gegenstände mit einem entsprechenden Kupplungsteil (10), das an einer durch ein Ventil (12) verschließbaren Be- und Entladeöffnung in einem trichterförmigen Oberteil (13) des Be- und Entladecontainers (11) angeordnet, dicht und tragfähig anschließbar ist.

2. Reinigungs- und Sterilisiermaschine nach Anspruch 1, **dadurch gekennzeichnet,** daß der Be- und Entladecontainer (11) mit seinem Kupplungsteil (10) an ein entsprechenden Kupplungsteil (20) einer zu einem Sterilraum (18) führenden Schleuse (19) einer Entlade- und Umfüllvorrichtung dicht und tragfähig über Kopf anschließbar ist, wobei in der durch einen Bodendeckel (21) verschließbaren Schleuse (19) kupplungsseitig Reinigungsund Sterilisierorgane (22) angeordnet sind.

3. Verfahren zum Reinigen und Sterilisieren kleiner Gegenstände, wie Verschlußelemente von pharmazeutischen Gefäßen, unter Verwendugn einer Reinigungs- und Sterilisiermaschine nach Anspruch 1 und 2, **dadurch gekennzeichnet,** daß nach Umfüllen der Gegenstände aus dem Container in den an dem Container dicht angekuppelten Behandlungsbehälter die Behandlung der Gegenstände in dem Behandlungsbehälter und parallel dazu die Reinigung und Sterilisierung des leeren Containers erfolgen und daß danach bei angekuppeltem Behandlungsbehälter die gereinigten und sterilisierten Gegenstände in den Container umgefüllt werden und nach Verschließen des Containers der Container von dem Behandlungsbehälter abgekuppelt und an der zu dem Sterilraum führenden Schleuse angekuppelt wird, über die dann nach Reinigung und Sterilisierung des abgeschlossenen Raums zwischen dem Ventil der Ent- und Beladeöffnung unter der Schleuse die gereinigten und sterilisierten Gegenstände in den Sterilraum entleert werden.

## Claims

1. A cleaning and sterilizing machine for small articles, such as closure elements for pharmaceutical containers, comprising a treatment vessel (1) which is mounted in a carrier (2) to pivot through 180 ° around a horizontal axis (3) and which has a funnel-shaped lower portion (4) comprising a closable discharge opening (5) and to which supply and discharge lines (7,8) for at least one treatment medium, such as water, steam or compressed air, are connected, characterized in that the discharge opening (5) can be closed by a valve (6) and is also constructed as a charging opening with a coupling member (9). Connectable in sealing-tight and supporting manner to said coupling member is a charging and discharge container (11) for the articles to be treated which has a matching coupling member (10) disposed at a charging and discharge opening closable by a valve (12) in a funnel-shaped upper portion (13) of the charging and discharge container (11).

2. A cleaning and sterilizing machine according to claim 1, characterized in that the charging and discharge container (11) can be connected inverted and in a sealing-tight and supporting manner via its coupling member (10) to a matching coupling member (20) of a lock (19), leading to a sterile room (18), of a discharge and transfer device, coupling side cleaning and sterilizing members (22) being disposed in the lock (19), which can be closed by a bottom cover (21).

3. A method of cleaning and sterilizing small articles, such as closure elements for pharmaceutical containers, using a cleaning and sterilizing machine as set forth in claims 1 and 2, characterized in that when the articles have been transferred from the container to the treatment vessel connected sealing-tight to the container, the articles are treated in the treatment container and the empty container is cleaned and sterilized parallel therewith, whereafter with the treatment vessel connected the cleaned and sterilized articles are transferred to the container, and after the container has been closed it is disconnected from the treatment vessel and connected to the lock which leads to the steril room and via which the cleaned and sterilized articles, after the cleaning and sterilization of the closed space between the discharge and charging opening and the lock, are emptied into the sterile room.

## Revendications

1. Machine de nettoyage et de stérilisation de petits objets, tels que des éléments de fermeture de récipients pharmaceutiques, constituée d'un récipient de traitement (1), monté pivotant de 180° autour d'un axe horizontal (3) dans un support (2) qui présente une partie inférieure (4) en forme d'entonnoir avec une ouverture de déchargement (5) pouvant être fermée, disposée dedans et à laquelle sont raccordées des conduites d'amenée et d'évacuation (7, 8) pour au moins un agent de traitement tel que de l'eau, de la vapeur, de l'air comprimé,
caractérisée en ce que l'ouverture de déchargement (5) peut être fermée par une valve (6) et est additionnellement réalisée sous forme d'ouverture de chargement avec une pièce d'accouplement (9), à laquelle un conteneur de chargement et de déchargement (11) pour les objets à traiter avec une pièce d'accouplement (10) correspondante qui est disposée sur une ouverture de chargement et de déchargement pouvant être fermée par une valve (12), dans une partie supérieure (13) en forme d'entonnoir du conteneur de chargement et de déchargement (11), peut être raccordé de manière étanche et capable de porter.

2. Machine de nettoyage et de stérilisation selon la revendication 1,
caractérisée en ce que le conteneur de chargement et de déchargement (11) peut être raccordé de manière étanche et capable de porter sur la tête, par sa pièce d'accouplement (10), à une pièce d'accouplement (20) correspondante d'un sas (19), conduisant à un espace stérile (18), d'un dispositif de déchargement et de transvasement, des organes de nettoyage et de stérilisation (22) étant disposés du côté accouplement dans le sas (19) pouvant être fermé par un couvercle de fond (21).

3. Procédé pour le nettoyage et la stérilisation de petits objets, tels que des éléments de fermeture de récipients pharmaceutiques, en utilisant une machine de nettoyage et de stérilisation selon la revendication 1 et 2,
caractérisé en ce que, après transvasement des objets hors du conteneur dans les récipients de traitement accouplés de manière étanche au conteneur, ont lieu le traitement des objets dans le récipient de traitement et parallèlement à cela le nettoyage et la stérilisation du conteneur vide et que, ensuite, avec le récipient de traitement accouplé, les objets nettoyés et stérilisés sont transvasés dans le conteneur et, après fermeture du conteneur, le conteneur est désaccouplé du récipient de traitement et accouplé au sas menant à l'espace stérile, par lequel, après nettoyage et stérilisation de l'espace isolé entre la valve de l'ouverture de chargement et de déchargement sous le sas, les objets nettoyés et stérilisés sont évacués dans l'espace stérile.
